# EUROPEAN PATENT APPLICATION

(11) **EP 2 243 825 A1**
(43) Date of publication of application: **27.10.2010**
(21) Application number: 09004795.2
(22) Date of filing: 01.04.2009
(51) Int. Cl.: C12N 5/071, G01N 33/50

(54) **Method for the generation of a skin model and skin model**

(71) Applicant: Universität zu Köln, 50923 Köln (DE)
(72) Inventor: Eckl, Katja Martina, 50658 Köln (DE); Hennies, Hans Christian, 50658 Köln (DE)
(74) Representative: Kröncke, Rolf

(57) **Abstract**

The present invention relates to a method for the in vitro generation of skin, in particular of a skin model. In a further aspect, the present invention relates to the skin model thus obtained and its use for screening candidate substances and pharmaceuticals.

## Description

The present invention relates to a method for the in vitro generation of skin, in particular of a skin model. In a further aspect, the present invention relates to the skin model thus obtained and its use for screening candidate substances and pharmaceuticals.

### Prior Art

A variety of skin-equivalent systems for in vitro use for research on skin transplants and grafts, for studies of the cellular and molecular biology but also for the cosmetical and pharmaceutical research have been developed. Recently, several disease specific models, either with patient samples or mimicking patients phenotype, have been described; e. g. Tjabringa G., 2008, Am. J. Pathol., 173:115-123. In the first cultures described, namely in conventional two dimensional tissue cultures, a feeder layer is provided, typically based on irradiated fibroblasts, whereon keratinocytes are seeded. Today three dimensional organotypic cultures are described, where the keratinocytes with or without mesenchymal cells, are cultivated at the air-liquid interface on various substrates serving as dermal equivalents. For example, three dimensional models mimicking severe disorders of keratinisation are described in Tjabringa supra; Harrison CA., et al, Br. J. Dermatol. 2007, 156:247-257. However, until today a model of keratinisation disorders for the study of barrier properties has not been described.

So far published as well as commercially available skin models show a hyperkeratosis of the stratum corneum over time, which increases dramatically from day 5. That is, one of the main drawbacks of all presently available skin models is the early development of hyperkeratosis, thus, preventing or limiting their use in pharmacological or cosmetical studies. Furthermore, they were not able to provide a stable skin model system allowing to study keratinisation disorders, particularly congenital keratinisation disorders, e. g. congenital ichthyosis. The hyperkeratosis observed in presently described skin models is likely to be caused by retention of the scales and other parameters influencing said skin model, like the culture medium, culture temperature, air pressure, CO₂ pressure and time of cultivation.

In recent years, several new genes for autosomal recessive congenital ichthyosis (ARCI) have been identified. ARCI, including lamellar ichthyosis (LI) and non-bullous congenital ichthyosiform erythroderma (NBCIE), forms a clinically and genetically heterogenous group of severe keratinisation discorders with a prevalence of 1 in 100.000 to 200.000 persons in the European and Northern American population. Although up to date six genes, namely, TGM1, ALOX12B, ALOXE3, ABCA12, ICHTHYIN (NIPAL4), and CYPF22 (FLJ39501), have been mapped, more loci must exist, as 30 - 40 % of all ARCI patients do not have mutations in one of these genes genes (Richard, 2004, Am J Med Genet 131C:32-44; Schmuth et al, 2007, Adv Dermatol 23:231-256). Only transglutaminase 1, 12R-LOX, and eLOX-3, the gene products of TGM1, ALOX12B, and ALOXE3, respectively, have been functionally analysed so far. Affected babies are often born wrapped in a parchment-like membrane, the collodion membrane. As this condition results in a massive disturbed water and mineral balance dysfunction, neonatal intense care is required, side effects may be ectropion, eclabion, as well as infections. After days to weeks the collodion membrane cracks leaving the skin more or less scaly with a mild to extensive underlying erythema. Color and adherence of scales vary from patient to patient, but depend also on body region, age, climate, treatment and race. Therapy so far is only symptomatic and mostly not sufficient. Side effects are hypohydrosis, secondary alopecia, itch and a social stigmatism with all its psychological problems.

12R-LOX and eLOX-3 act as subsequent members of the same pathway, and convert arachidonic acid via 12R-hydroperoxyeicosatetraenoic acid (12R-HPETE) to the corresponding hepoxilin-like epoxyalcohol, 8R-hydroxy-11R,12R-epoxyeicosatrienoic acid. Mutations in ALOX12B and ALOXE3, the genes for 12R-LOX and eLOX-3, were found in approx. 14 % of all patients with autosomal recessive congenital ichthyosis (ARCI) (18;37) (Eckl et al.. J Invest Dermatol. 2009 Jan 8).

In order to gain a patient independent human model mimicking the barrier defect and hyperkeratosis typical for e. g. ARCI, there is a need for a three dimensional skin model testing of potential candidates for therapy of said disease. In this connection, a major challenge for finding a suitable three dimensional skin model for keratinisation disorders is the thickening of the stratum corneum typically seen over time without a genetic defect. To evaluate therapeutics with a skin model, it must develop a basal layer attached to the dermal layer and show a normal differentiation pattern. Further, to evaluate therapeutics for specific skin diseases, the skin model must be close to the "natural disease". Further, the skin model must reflect the natural situation of normal skin without developing hyperkeratosis at an early time point, thus, falsifying the results.

Hence, an object of the present invention is to provide a method for the in vitro generation of a skin model of normal skin as well as a skin model for skin disorders, like keratinisation disorders. Another object of the present invention is to provide a skin model in particular suitable for performing pharmaceutical and cosmetical studies.

### Brief description of the invention

The present inventors aim to develop a skin model overcoming the above drawbacks. Namely, the present inventors developed a method for in vitro generation of a skin model providing a skin model for normal skin as well as a skin model for skin disorders, like keratinisation disorders, in particular, ARCI.

In a first aspect, a method is provided for in vitro generation of a skin model comprising the steps of:
- preparing a matrix in a first permeable container whereby said matrix contains collagen I, culture medium having a neutral pH, and primary fibroblasts and whereby the first container is inserted in a second container in a way that the permeable bottom of the first container is spaced apart from the bottom of the second container;
- storing the container in an incubator for 1 to 6 hours, preferably, 1 to 3 hours;
- adding a culture medium to the first and second container;
- incubating the container in an incubator with CO₂ enriched atmosphere at high humidity for 1 to 6 hours;
- removing the culture medium from the top of the matrix in the first container;
- adding primary keratinocytes in culture medium onto the matrix;
- incubating the container for 12 to 36 hours, preferably 20 to 26 hours;
- removing the culture medium from the matrix present in the first container;
- exchanging the medium in the second container whereby the level of the culture medium in the second container is below the upper surface of the matrix present in the first container;
- further cultivation of the cell while exchanging the culture medium every second day
whereby the fibroblasts and keratinocytes are derived from the same donor and the primary keratinocytes and primary fibroblasts are obtained from passages 1 to 5.

Typically, a skin model is obtained after seven days total cultivation.

In a further aspect, a skin model is provided reflecting a normal skin or, if modified accordingly, reflecting a skin disorder, like ARCI. Following the specific process steps as identified in the main claim, it was able to obtain a skin model wherein the problem of hyperkeratosis does not develop within the first ten days. In addition, in another aspect, a skin model system is provided mimicking congenital ichthyosis or other keratinisation disorders. Further, a method for screening of candidates for the treatment of a keratinisation disorder is provided using the skin model according to the present invention. Finally, the present invention provides a method for determining the regimen for treating an individual suffering from a keratinisation disorder using a skin model built up with cells obtained from said individual.

That is, the model according to the present invention can be used for pharmaceutical studies, e. g. substitution studies of known genetic defects occurring in various keratinisation disorders as well as in identifying pharmaceuticals, like therapeutics, for said disorders.

Moreover, the skin model allows determining adverse side effects of potential or known pharmaceuticals or cosmetics, thus, avoiding animal testing.

### Brief description of the drawings

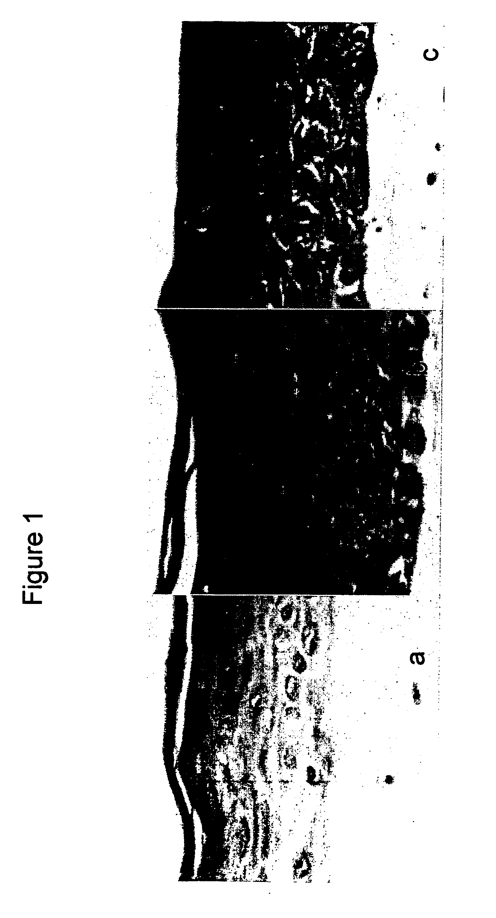

Fig.1:a Model after 25 d of cultivation at the air liquid interface in SKDM (H/E staining). b-c:SKDM medium was supplementated with 10ng/ml TGFβ 1. H/E staining of 3D skin models cultivated in SKDM/TGFβ 1 showed a more or less prominent hyperkeratosis, flatten basal keratinocytes, disorganisation of the epidermis and detachment from the underlying dermal matrix. d: This example shows a skin model prepared with commercially purchased normal adult foreskin keratinocytes. Note unexpected thinckening of the SC. e-f: Change of differentiation medium to KCM resulted in 3D skin models shown here. Note upright basal keratinocytes and a normal stratum corneum. A hyperkeratosis is not visible. For all figs: magnification 200x.

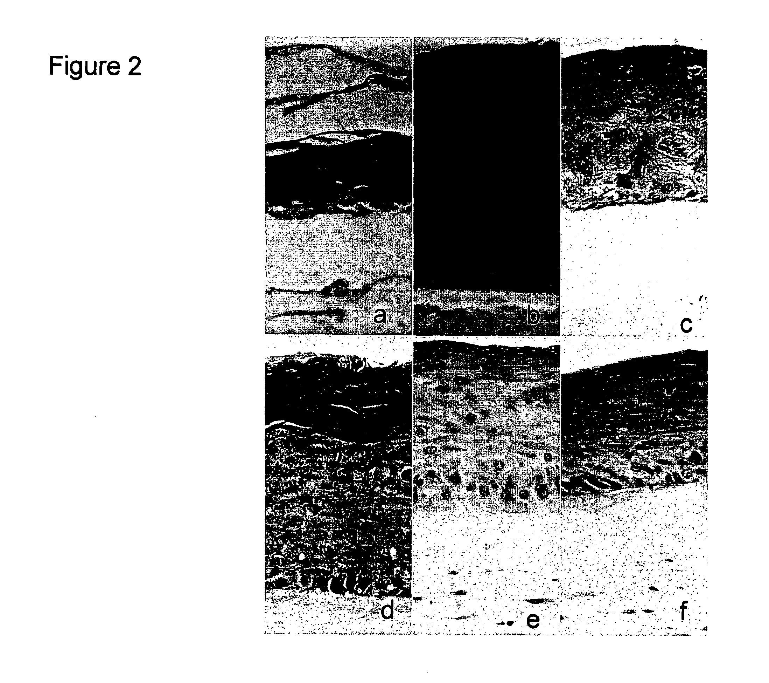

Fig. 2a-c: Skin models mimicking congenital ichthyosis (H/E staining). To mimic ARCI, specific genes have been seletively knocked down using siRNA technology. Shown here are models with knock down in ALOX12B (2a), ALOXE3 (2b) and TGM1 (2c), magnification was 200x.

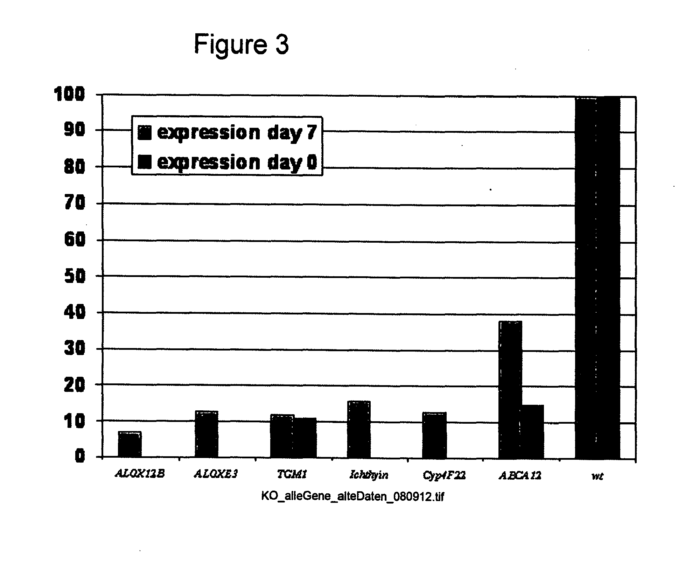

Fig. 3: Summary of semi-quantitative expression studies for single knocked down genes in skin models (grey bars) after 7 days of incubation at the air liquid interface as well from keratinocytes (black bars) at time point zero. Expression was normalised against models without knock out (wildtype, wt) as well as against a housekeeping gene (RPS18) for all samples.

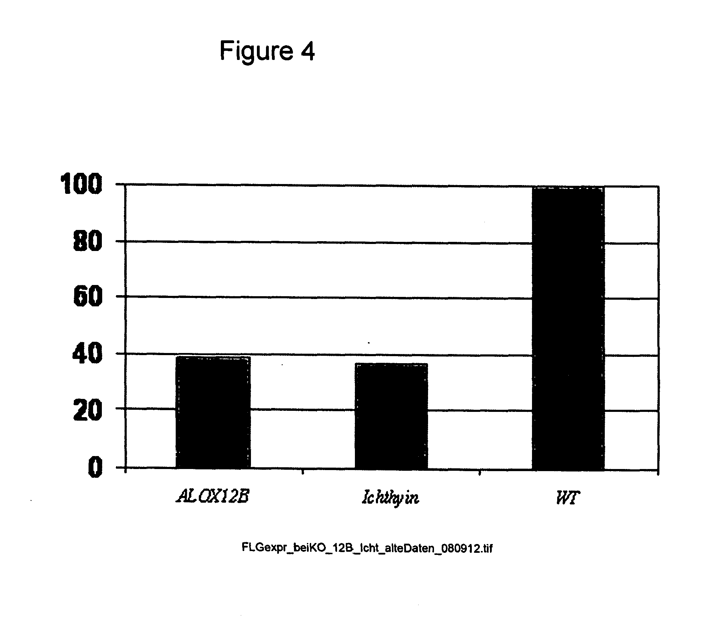

Fig. 4: Expression of filaggrin (FLG) is significantly reduced in skin models with knock down in ALOX12B or Ichthyin.

Fig. 5: In Fig.5 a typical example of the methods steps according to the present invention is shown.

### Detailed description of the present invention

In a first aspect, a method for the in vitro generation of a skin model is provided. The method comprising the steps of:
A method for the in vitro generation of a skin model comprising the steps of:
   - preparing a matrix in a first permeable container whereby said matrix contains collagen I, culture medium having a neutral pH, and primary fibroblasts and whereby the first container is inserted in a second container in a way that the permeable bottom of the first container is spaced apart from the bottom of the second container;
   - storing the container in an incubator for 1 to 6 hours, preferably, 1 to 3 hours;
   - adding a culture medium to the first and second container;
   - incubating the container in an incubator with CO₂ enriched atmosphere at high humidity for 1 to 6 hours;
   - removing the culture medium from the top of the matrix in the first container;
   - adding primary keratinocytes in culture medium onto the matrix;
   - incubating the container for 12 to 36 hours, preferably, 20 to 26 hours;
   - removing the culture medium from the matrix present in the first container;
   - exchanging the medium in the second container whereby the level of the culture medium in the second container is below the upper surface of the matrix present in the first container;
   - further cultivation of the cell while exchanging the culture medium every second day
   whereby the fibroblasts and keratinocytes are derived from the same donor and the primary keratinocytes and primary fibroblasts are obtained from passages 1 to 5.

The term "high humidity" as used herein refers to a humidity of the air of at least 80%, e.g. 85%, like 90%, preferably 95%.

CO₂ enriched atmosphere refers to an atmosphere were the CO₂ concentration is above the CO₂ concentration in natural air. For example, the CO₂ concentration is above 1%, like above 2%, 3%, 4%, or 5%. A CO₂ concentration of 5% is typically present in incubators for cell culture.

In a preferred embodiment, the primary fibroblasts and the primary keratinocytes are used for the generation of the skin model are obtained from the same cultivation passage, in particular, from passage 2, 3 or 4 of cultivation. Preferably, the keratinocytes where grown without a feeder layer in a cell free system before until addition to the matrix for skin model preparation.

The matrix is preferably prepared with serum, e.g. foetal calf serum (FCS). According to the method of the present invention, a fully developed skin model is obtained after seven days total cultivation. Preferred, the medium wherein the keratinocytes are added to the matrix is a serum-free medium. The culture medium is preferably KGM medium (Lonza, Rockland), containing 0,15 mM Calcium. The medium KGM is described e.g. in Nickoloff, 1988, Am J Path, 132(3):543-551;

The medium used for cultivation is preferably the KCM medium as described by Allen-Hoffman and Rheinwald, 1984, Proc Natl Acad Sci USA, 81:7802-7806, that is, the KCM medium is used after the first medium change, namely when initiating differentiation of the cells after removal of the culture medium from the first container. KCM induces keratinocytes in the absence of feeder cells and high cell densities to differentiate, due to the high Ca²⁺ concentration (1.2 mM), the presence of serum (10%) but a balance between proliferation and differentiation can be held, as cholera toxin and EGF support proliferation and basal keratinocyte maintenance as well as hydrocortisone helps to improve cell and epidermal tissue layer architecture (Green, Cell 15(3):801; 1978 and Rheinwald and Green, Cell 6(3):331; 1975).

In a preferred embodiment, the primary fibroblasts are seeded in a density of from 1X10⁴ to 1x10⁶ cells/cm³ matrix, preferably of from 3x10⁴ to 5x10⁵ cells/cm³ matrix, like 9x10⁴ cells/cm³ matrix.

For the primary keratinocytes, the density is preferably of from 3x10⁵ to 7x10⁶ cells/cm² matrix surface, e. g. 8x10⁵ to 3x10⁶ cells/cm² suface of the matrix, like 1x10⁶ to 2x10⁶ cells/cm² matrix sufrace.

In addition, it is preferred no TGFβ 1 is added to the culture medium in contrast to the general teaching in the prior art. The present inventors found that in contrast to other reports TGFβ 1 diminishes the normally large quantity of basal cell proliferation.

The KCM medium has the composition as shown in table 1 below

**Table 1: KCM medium**

| Component | absolute | for 500 ml |
|---|---|---|
| DMEM | 64.5% | 322.5 ml |
| Ham's F-12 | 21.5% | 107.5 ml |
| FCS | 10% | 50 ml |
| Hydrocortisone | 0.4 µg/ml | 5 ml |
| Cholera Toxin | 10⁻¹⁰ M | 50 µl |
| EGF | 10 ng/ml | 5 ml |
| Insulin | 5 µg/ml | 500 µl |
| Glutamine | 4 mM | 10 ml |

The present inventors found that in contrast to most studies from recent years, the differentiation of the keratinocytes into the skin model system occurs not in a serum-free medium but in a serum-containing full medium. Furthermore, the present inventors found that it is preferred to provide specific densities of each of the cell types, namely primary keratinocytes and primary fibroblasts. Moreover, an important aspect of the present invention is the use of primary keratinocytes and primary fibroblast obtained from the same donor.

It is important to obtain the primary fibroblast and keratinocytes at passages not later than passage five, preferably obtained from passage 2 or 3. Moreover, removing the culture medium from the first container induces a differentiation of the cells present in the matrix formed from collagen I containing serum, like FCS. The differentiation is induced by the change of medium to the KCM medium as defined above and the shift to the air liquid interface one day after model preparation. The density of the keratinocytes in normal human eidermis is 3x10⁶ cells/cm².

The present inventors found that according to the method of the present invention it is possible to obtain skin models not suffering from the problem of an early hyperkeratosis, e.g. at day 5 of cell culture. The skin model according to the present invention does not show hyperkeratosis before day 10, preferably not before day 14 of cultivation. Thus, the barrier formations remain unchanged during cultivation.

In another preferred embodiment, the generated skin model represents a skin model for a keratinisation disorder, e. g. for ARCI. ARCI is a rare and severe keratinisation disorder. ARCI is a monogenic disorder. Today mutations in six genes are known to be associated with ARCI, but only little is known about the function, metabolism and interaction of all these gene products. According to the present invention, to mimic congenital ichthyosis, each of the known six ARCI causing genes were knocked down in primary human keratinocytes seeded in a two dimensional cultured system prior to adding said primary keratinocytes to the three dimensional skin model preparation claimed herein. Namely, a siRNA where introduced silencing or knocking down the genes TGM1, ALOX12B, ALOXE3, ABCR12, ICHTHYIN(NIPAL4) and CYPF22. The respective GenBank Accession Nos. are shown in table 2.

Preferably, the primary keratinocytes are treated with the silencer RNA for the respective genes 20 to 36 hours in advance, preferably, 22 to 28 hours in advance before adding said keratinocytes to the matrix composed of collagen I and primary fibroblasts. Using a method according to present invention with keratinocytes incubated with siRNA as described above in advance, it was possible to obtain a three dimensional skin model for congenital ichthyosis. It was verified that said skin model is usable as a model for congenital ichthyosis based on a Lucifer Yellow assay. Said Lucifer Yellow assay demonstrated the disturbed barrier function known for congenital ichthyosis. This is the first time, a dye penetration assay, namely the Lucifer Yellow assay, is described to prove the barrier function in skin model systems.

For pharmacological studies media containing serum (e. g. 10 % foetal calf serum) may be used although said serum may contain not only required elements for allowing differentiation but also other compounds which may influence the model e. g. by inducing immunological side effects. However, it has been shown that the side effects can be neglected since no adverse side effects were observed during cultivation.

For other known monogenic or oligogenic based keratinisation disorders, the skin model according to the present invention may be used accordingly. That is, starting from siRNA for the known genes causing the keratinisation disorder, the primary keratinocytes may be prepared in the respective culture media and, afterwards, are used for generating the three dimensional skin model.

The containers used in the method according to the present invention are containers known in the art for preparing three dimensional organocultures. It is necessary that the matrix present in the first container can exchange compounds with the medium present in the second container via a permeable membrane present in the first container.

In a further aspect, the present invention relates to a method for screening candidates for dymptomatic or causal treatment of keratinisiation disorders using the skin model according to the present invention. The method comprises the step of providing candidate compounds, contacting said candidate compounds with the skin model for a keratinisation disorder according to the present invention, assaying for a change of physiological or morphological properties of the skin model and identifying candidates causing the change of the physiological or morphological properties, thus, representing a candidate molecule for preventing signs of disease or treating said characterisation disorder.

In a further aspect, the present invention relates to a method for assaying the therapeutical or cosmetical potential of a candidate compound comprising the step of contacting said candidate compound with the skin model derived from primary normal cells according to the present invention and assaying for a change in the physiological or morphological properties of said model to identify any positive or adverse effects in the skin model.

The skin model is also particularly useful for determining adverse effects of new or known compounds, e. g. determining toxicity of said compounds. This is in particular necessary for compounds used as therapeuticals or in cosmetic compositions.

The compounds to be tested may be compounds known as small molecules and may be compounds typically present in cosmetics or pharmaceuticals.

Physiological or morphological changes or properties may be assayed by known methods. For example, the Lucifer Yellow assay may be used to assay the barrier function of the skin.

Further, the model may be used to investigate candidate compounds, e. g. genes or gene products as well as metabolites whose involvement is presumed in a specific keratinisation disease or disorder.

That is, the present invention provides a tool for assessing therapeutic approaches for keratinisation disorders. Thus, the skin model according to the present invention represents a screening tool for therapeutic or cosmetic compounds useful in keratinisation disorders or useful in cosmetics.

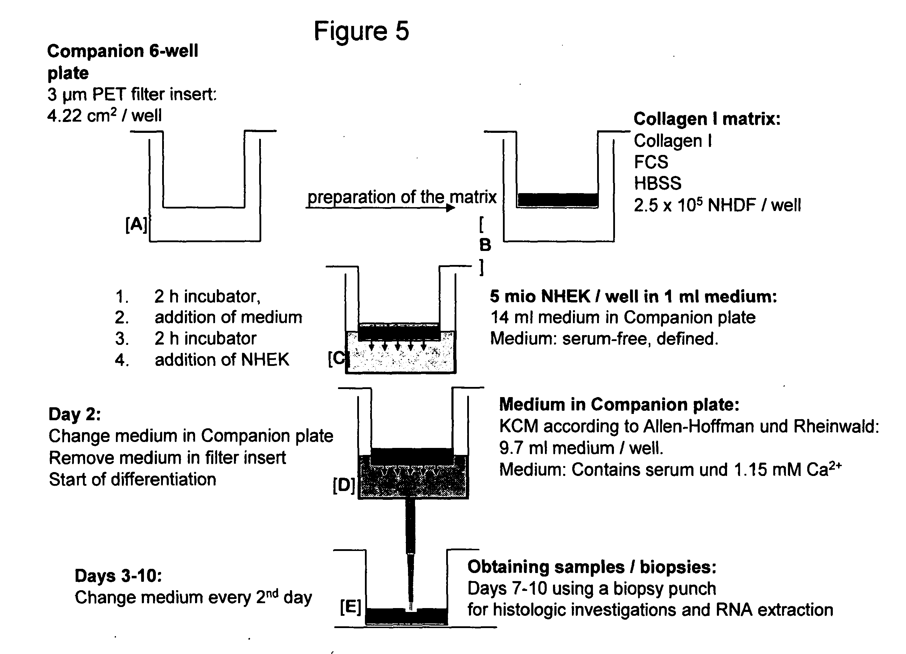

The method according to the present invention will be described in more detail referring to the flowchart of fig. 5.

In Fig.5 a typical example of the methods steps according to the present invention is shown. Namely, a first and a second container are provided, e.g. a companion 6-well plate whereby the first container, the insert, has a permeable filter at the bottom, to allow an exchange of compounds between the matrix present in the insert and the medium present in the second container. In a first step, the matrix is prepared, e.g. by admixing collagen I with FCS, 10x HBSS and primary fibroblasts, e.g. NHDF (primary human foreskin fibroblasts). The matrix is allowed to settle in the first container while stored for 2 h in an incubator at normal air. After 2 h medium, e.g. KGM medium is added onto the matrix. In addition, serum-free culture-medium, KGM, is added into the second container, in a way that the matrix is immersed into said medium present in the second container and the matrix is further incubated for another 2 hours in an incubator at high humidity having a CO₂ concentration of 5 % at 37°C. Thereafter, the primary keratinocytes, NHEK (primaryhuman foreskin keratinocytes) derived from the same donor and obtained from the same culture passage, which may optionally be modified as described herein, are added onto the matrix.

The system is incubated further in an incubator for about 24h. At day 2, the medium is changed in the second container, the Companion plate, while the medium in the first container is removed. Thus, an air-liquid interface is given for the matrix. The medium used for further cultivation is KCM medium as described herein. Said medium contains serum and 1.15 mM Ca²⁺. Starting from day 2, the medium. KCM, is changed every second day. After 7 days culture in total, a skin model according to the present invention is obtained. Said skin model may be used as a screening tool or as a model system for in vitro investigations.

In the following, the present invention will be explained by examples in more detail. It is understood that said examples do not limit the present invention.

### Examples:

### MATERIALS AND METHODS

### Media and Solutions

**KCM** (Keratinocyte Culture Medium) was first described by Allen-Hoffmann and Rheinwald, Proc Natl Acad Sci USA, 1984, 81:7802-7806 and is prepared as follows:

### A. Buffers and Solutions

### 1. HBES

| | |
|---|---|
| HBSS | 95 ml |
| HEPES | 5 ml |

| | |
|---|---|
| Store at 2-8°C (up to 24 months) | |

### 2. 0.005 N HCI

Prepare 0.5 M HCl Fixanal® (standard solution) and dilute 1:100 with sterile water (cell culture pure). Sterile filter prepared 0.005 N HCI solution.

### 3. 5 N NaOH

Dissolve 20 g NaOH in 100 ml ice cold sterile water (cell culture pure) and store in a glass bottle at RT.

### B. Stocks

### 1. Hydrocortisone

| **1a.** | |
|---|---|
| Hydrocortisone | 1 g |
| Ethanol absolute | 200 ml |

| | |
|---|---|
| Hydrocortisone/EtOH (5mg/ml) stored at -20°C. | |

**1b**

| | |
|---|---|
| Hydrocortisone/EtOH (5 mg/ml) | 800 µl |
| HBES | 100 ml |

| | |
|---|---|
| Aliquot à 10 ml and store at -20°C. | |

**2. Epidermal Growth Factor (human recombinant)**

| | |
|---|---|
| EGF | 200 µg |
| DMEM* | 200 ml |

| | |
|---|---|
| Aliquot à 10 ml and store at -20°C. *DMEM with 4500 mg/L glucose, without glutamine and without pyruvate. | |

**3. Cholera Enterotoxine (from Vibrio Cholerae)**

| | |
|---|---|
| Cholera Enterotoxin | 1 mg |
| Sterile Water | 10 ml |

| | |
|---|---|
| Aliquot à 1 ml and store at -20°C. | |

**4. Insulin (bovine pancreas)**

| | |
|---|---|
| Insulin | 250 mg |
| 0.005 M HCl | 50 ml |

| | |
|---|---|
| Prepare and aliquot à 0.5 ml. Store at -20°C. | |

**5. Adenine (base)**

| | |
|---|---|
| Adenine | 243.2 mg |
| NaOH (5 N) | 2 ml |
| Sterile Water | ad 10 ml |

| | |
|---|---|
| Add NaOH first, stir, then add water, stir using a magnetic bar. Heat if necessary. When fully dissolved, aliquot à 0.5 ml and store at -20°C. Heat when thawing using a water bath to resolve adenine fully. | |

### C. Media

**2. KCM**

| Component | absolute | 500 ml |
|---|---|---|
| DMEM | 64.5% | 322.5 ml |
| Ham's F-12 | 21.5% | 107.5 ml |
| FBS | 10% | 50 ml |
| Hydrocortisone | 0.4 µg/ml | 5 ml |
| Cholera Toxin | 10⁻¹⁰ M | 50 µl |
| EGF | 10 ng/ml | 5 ml |
| Insulin | 5 µg/ml | 500 µl |
| Glutamine | 4 mM | 10 ml |
| Adenine | 0.00018 M | 500 µl |

| | | |
|---|---|---|
| Store at 2-8°C up to 2 weeks. | | |

**C. List of Suppliers**

| | | | |
|---|---|---|---|
| HBSS (w/o Ca, Mg, phenol red) | 14175053 | 500 ml | Invitrogen |
| HEPES 1 M | 15630056 | 100 ml | Invitrogen |
| DMEM | 11960044 | 500 ml | Invitrogen |
| Ham's F-12 | 31765027 | 500 ml | Invitrogen |
| Sterile water (cell culture pure) | 15230089 | 500 ml | Invitrogen |
| FBS | 10270106 | 500 ml | Invitrogen |
| (EU approved, from South America, not heat inactivated) | | | |
| Insulin (bovine pancreas) | 15500-250MG | 250 mg | Sigma-Aldrich |
| Adenine (free base) | A2786-5G | 5 g | Sigma Aldrich |
| Cholera Toxin | C8052-1MG | 1 g | Sigma Aldrich |
| Epidermal Growth Factor | E9644-.2MG | 0.2 mg | Sigma Aldrich |
| Hydrocortisone | H0888-1G 1 g | | Sigma Aldrich |
| Sodium hydroxide (ultrapure) | S8045-1Kg | 1 kg | Sigma Aldrich |
| Fixanal® ( 0.5 N HCI) | 38285-1EA | 0.5 mol | Sigma Aldrich |

**SKDM** with ascorbic acid, transferrin, BSA fatty acid free in KGM without vovine brain extract was prepared according to Mildner et al., 2006, supra.

**Ultrapure bovine Collagen** I for tissue engineering was ordered from Nutacon (Leimuiden, The Netherlands)

**Lucifer Yellow** was purchased from Sigma, a 1mM working solution was prepared in 1x PBS (w/o Ca²⁺/Mg²⁺) and stored in the dark.

Meyer's Hematoxiline and Eosin Y (in water) were purchased from Carl Roth (Karlsruhe, Germany).

**Cell culture grade chemicals** have been purchased from either Sigma Aldrich, Invitrogen or Lonza, as indicated in the text. KGM is a trademark from Clonetics, Lonza (Basel, Switzerland).

### TGFβ1 was from BD Biosciences (Heidelberg, Germany).

**Primary antibodies** against human collagen IV (ab6586, rabbit), cytokeratin 5 (ab17130, mouse), cytokeratin 10 (ab53124, mouse), filaggrin (ab24584, mouse), integrinß 1 (ab3167, mouse) were all from Abcam (Cambridge, UK), antibody angainst human involucrin (GTX17810, mouse) was from GeneTex (San Antonio, TX, USA). Fluorescence labeled secondary antibodies were Texas Red goat anti mouse (T862) and Texas Red goat anti rabbit (T2767, both Molecular Probes, Invitrogen, Karlsuhe, Germany)

### Isolation and culture of NHEK and NHDF

Primary human keratinocytes were isolated from neonatal foreskin and cultivated until second passage either in the presence of a feeder layer of irritated 3T3 mouse fibroblasts as described in detail by Navsaria and colleagues in Keratinocyte methods (Irene leigh and Fiona Watt, 1994, Cambridge University Press, p. 17-12) or without feeder cells under serum free low calcium conditions as described by Mitra and Nickoloff in Keratinocyte Methods (Irene Leigh and Fiona Watt; 1994, Cambridge University Press, p. 17-19). Passage 2 keratinocytes were cryopreserved until cultivation for 3D model preparation. Cultivation was then performed in serum free, defined cultivation media with low calcium concentrations (KGM, Lonza). Additionally some keratinocyte stocks have been purchased from Lonza and have been maintained in KGM until 3D model preparation according the manufactors recommendations. NHDF have been isolated from neonatal foreskin as described by Navsaria et al. and cultivated in DMEM with 10 % FCS, 100 IU/ml penicillin and 100 µg/ml streptomycin.

### 3D Model preparation

Tissue engineering was performed similar to Smola and colleagues, 1998, Exp.Cell.Res., 239:399-410 and Mildner et al. (supra) using 6 well deepwell plates with 3 µm insert with 4,22 cm² growtharea (BD biosciences) . A mixture of Collagen I (bovine, 3mg/ml) and 10xHBSS was brought to neutral pH with 2N NaOH, 2,5 x 10⁵ NHDF in FCS were added and the mixture was poured into the filter inserts (2,5 ml each). The matrix was stored for 2-4 h in a CO₂ free incubator before KGM medium was added to the system (2 ml onto the matrix, 14 ml around the filter insert, each). The system was transferred to an incubator with 5% CO2, 95 % humidity. After removing the medium from the top of the matrix 2-4 hours later 4,5 to 5,2 x 10⁶ NHEK in 1 ml KGM/per well were seeded onto the matrix. At the next day the keratinocytes have been settled and the medium inside the insert was removed. Medium around the insert was exchanged for SKDM (Mildner et al, supra) or KCM or special preparations as mentioned below as differentiation medium. Models were cultivated for 7 to 21 days with change of medium every second day. 3D skin model samples were then harvested using biopsy punches (5 to 8 mm diameter). Samples were either processed and embedded in paraffin, cryofrozen or RNA and/or Protein was isolated. For origin, passages and combination of NHEK with NHDF used see results.

### Knock down of ARCI genes in keratinocytes with siRNA

NHEK in 2D cultures were grown under feeder free, serum free conditions up to 40% confluency. Knock down with Stealth® select siRNA (Invitrogen) was then performed according Mildner et al. 2006 (supra) with some modifications, in brief: for each T75 flask 1300 pmol of each siRNA were diluted in a total of 1,5 ml Opti-MEM. In parallel 50 µl of Lipofectamine 2000 (Invitrogen) was mixed with 1,5 ml Opti-MEM.

Both solutions were combined, and after incubation at RT, poured onto the keratinocytes. Cells were harvested after 24 h hours and used as well for 3D model preparation and for semi quantitative RT PCR analysis to determine knock down. Knock down was performed for genes *ALOX12B, ALOXE3, TGM1, CYP4F22, ABCA12* and *ICHTHYIN (NIPAL4),* see table 3 for further details of the RNAi sequences.

### Semi quantitative RT PCR

To investigate knock down efficiency samples were taken at day zero (keratinocytes 24 h post siRNA transfection) and at day 7 to 8 (3D skin model sample after full differentiation of the epidermis, 192 to 216 h post siRNA transfection). Total RNA was isolated using standard techniques (including DNA digesting). RNA was normalized to 10 ng/µl and used for semi quantitative RT-PCR using a LightCycler 480 system (LightCycler® 480 RNA Master Hydrolysis Probes Kit, Roche Diagnostics - Applied Sciences, Mannheim, Germany). Normalisation was done with the housekeeping gene RPS18 (NM_022551). Primer and Probes for all assays are listed in table 2.

### H/E staining and Immunostaining

Models were harvested using a biopsy punch (5-8 mm in diameter), cut diagonally, fixed in formalin, processed and embedded in paraffin. 5µm sections were cut followed by H/E staining or immunostaining. For H/E staining samples were deparaffinisated, hydrated, stained, rehydrated and mounted with Gurr (BDH, VWR, Darmstadt, Germany). For immunostaining cuts were deparaffinisated, hydrated and cooked in a microwave oven for 10 min in citric puffer (ph 6) for antigen retrieval. After cooling down for 20 min slides were washed, and blocked for 2 h at RT. Primary antibody incubation was performed over night at 4°C in a humidified chamber, secondary antibody incubation was done at RT for 3 to 4 hours in a humidified chamber in the darkness as secondary antibodies were fluorescence labelled. After secondary antibody incubation counter staining with DAPI (Sigma, 1ng/µl in PBS) for 5 min at RT was performed. Slides were then washed and mounted with polyvinyl alcohol mounting medium (Fluka, Sigma). Samples were visualized with a LMD6000 (Leica, Wetzlar, Germany).

### Lucifer Yellow Diffusion Assay

Lucifer Yellow Diffusion Assay was performed as describe earlier by Matsuki and colleagues, 1998, Proc Natl Acad Sci USA, 95:1044-1049 and Epp and co-workers, 2007, J Cell Biol, 177:173-182 with slide modifications to adapt to our system: At day 7 medium was exchanged and a liquid but not hot mixture of paraffin, vaseline and linoleic acid (VALAP, as described by Andrew and colleagues, Basic Methods in Microscopy, edited by Spector and Goldman, 2006, Cold Spring Harbor Laboratory Press, p. 1005-124) was used to define the epidermal area and separate it from non-populated areas of the insert. This was done using a 1 ml syringe. Inside the VALAP ring 500 µl of a 1 mM Lucifer Yellow solution (prepared in 1x PBS) was poured. The system was transferred to a humidified incubator for 1h at 37°C. After incubation samples were carefully washed with 1x PBS, cryofrozen and cut in 7 µm slices. Cuts were counterstained with Dapi (1µg/ml) and visualized using a LMD6000.

**Table 2**

| **Gene** | **Accession No.** | **Primer I** | **Probe (Roche LightCycler® 480 Assay)** |
|---|---|---|---|
| *ALOX12B* | NM_001139 | F Seq. ID No.1 R Seq. ID No.2 | ACC CGA GGG CAA GAT GAT GCA GGA AGA TGG GGC AAT Probe 42 supplied by Roche |
| *ALOXE3* | N_0021628 | F Seq. ID No.3 R Seq. ID No.4 | AAG CTT CGA GGG CTG TTG CAC CAG TGC TCT GTG ACA TAC TT Probe 42 supplied by Roche |
| *CYP4F22* | NM_173483 | F Seq. ID No. 5 R Seq. ID No.6 | AGG GGA AGA CCT TGG ACT TTA CGT CTG ACA GTT CCT TTC CAT Probe 68 supplied by Roche |
| *NIPAL4 [ICHTHYIN (v5)]* | N_001099287 | F Seq. ID No.7 R Seq. ID No.8 | CAA CAC CAG CTG CGA GAA C TGA CAA TCT GGC ACA GGA CT Probe 68 supplied by Roche |
| *TGM1* | NM_000359 | F Seq. 10 No.9 R Seq. ID No.10 | TGA ACC ATG ATT CTG TCT GGA ACC TGC CAC CCA TCA AAG Probe 42 supplied by Roche |
| *ABCA12* | NM_173076 | F Seq. ID No.11 R Seq. ID No.12 | ACT GCA CTG GCC TTG ATA GG TCG ACT TCG GAT CCA TGC Probe 42 supplied by Roche |
| *RPS18 [ribosomal protein S18]* | NM_022551 | F Seq. ID No.13 R Seq. ID No.14 | TGC GAG TAC TCA ACA CCA ACA GCA TAT CTT CGG CCC ACA Probe 70 supplied by Roche |

**Table 3**

| **Gene** | **Accession No.** | **Seq. ID No.** | **RNAi sequences from 5' to 3' (Invitrogen Stealth™ Select RNAi, set of 3) TOP BOTTOM** | | **Seq.ID No** |
|---|---|---|---|---|---|
| *ALOX12B* | NM_001139 | 15 | AGA AGG CCU CAG CAA UGA GGU GUG U | ACA CAC CUC AUU GCU GAG GCC UUC U | 16 |
| | | 17 | AAA GUG GUU CAG CAG CUG CUU AUG G | CCA UAA GCA GCU GCU GAA CCA CUU U | 18 |
| | | 19 | UAG UUG CAG UAC CAA GGG UCC UUG G | CCA AGG ACC CUU GGU ACU GCA ACU A | 20 |
| *ALOXE3* | NM_0021628 | 21 | UAC UUU GUC GUG AAG GUC UUA UGG C | GCC AUA AGGA CCU UCA CGA CAA AGU A | 22 |
| | | 23 | AGA AAU UGG UGU AGG UGA AGU GGG C | GCC CAC UUC ACC UAC ACC AAU UUC U | 24 |
| | | 25 | UUG CUA ACC AAC CAG AAG AGG AGG A | UCC UCC UCU UCU GGU UGG UUA GCC A | 26 |
| *CYP4F22* | NM_173483 | 27 | AAU UGA ACA GCA UCC AAG AGA UCC C | GGG AUC UCU UGG AUG CUG UUC AAU U | 28 |
| | | 29 | UGA CCA AGC AGA UGA UUC CUU UGG G | CCC AAA GGA AUC AUC UGC UUG GUC A | 30 |
| | | 31 | AAC AUA UCA AGG GAG ACC GCU GAG C | GCU CAG CGG UCU CCC UUG AUA UGU U | 32 |
| *NIPAL4 [ICHTHYIN (v5)]* | NM_001099287 | 33 | AAA GCA UGC AGC AUG AAC ACG CCC A | UGG GCG UGU UCA UGC UGC AUG CUU U | 34 |
| | | 35 | AGG AAU GGA UUA UAA AUA CUU UGG G | CCC AAA GUA UUU AUA AUC CAU UCC U | 36 |
| | | 37 | AGC AGG UUC AGA CUC UCC CUC AGG A | UCC UGA GGG AGA GUC UGA ACC UGC U | 38 |
| *TGM1* | NM_000359 | 39 | UAU AGA GGU AGG UGA UGU CCU CCC G | CGG GAG GAC AUC ACC UAC CUC UAU A | 40 |
| | | 41 | UUC ACU ACU AGC AUG CCC UCU GGG A | UCC GAG AGG GCA UGC UAG UAG UGA A | 42 |
| | | 43 | UUC AGA UUC UGC CCA CUG GCC UUG A | UCA AGG CCA GUG GGC AGA AUC UGA A | 44 |
| *ABCA12* | NM_173076 | 45 | UAA ACA UGA GGC CAU UGC UCU UCU C | GAG AAG AGC AAU GGC CUC AUG UUU A | 46 |
| | | 47 | UUC AGA AGC AUC UGC ACU GUU AUU G | CAA UAA CAG UGC AGA UGC UUC UGA A | 48 |
| | | 49 | AAU AAU CUC AGC UCU AUU AUC AGG G | CCC UGA UAA UAG AGC UGA GAU UAU U | 50 |

## Claims

1. A method for the in vitro generation of an skin model comprising the steps of:
- preparing a matrix in a first permeable container whereby said matrix contains collagen I, culture medium having a neutral pH, and primary fibroblasts and whereby the first container is inserted in a second container in a way that the permeable bottom of the first container is spaced apart from the bottom of the second container;
- storing the container in an incubator for 1 to 6 hours;
- adding a culture medium to the first and second container;
- incubating the container in an incubator with CO₂ enriched atmosphere at high humidity for 1 to 6 hours;
- removing the culture medium from the top of the matrix in the first container;
- adding primary keratinocytes in culture medium onto the matrix;
- incubating the container for 12 to 36 hours;
- removing the culture medium from the matrix present in the first container;
- exchanging the medium in the second container whereby the level of the culture medium in the second container is below the upper surface of the matrix present in the first container;
- further cultivation of the cell while exchanging the culture medium every second day
whereby the fibroblasts and keratinocytes are derived from the same donor and the primary keratinocytes and primary fibroblasts are obtained from passages 1 to 5.

2. The method according to claim 1 **characterized in that** the primary fibroblasts and the primary keratinocytes are obtained from the same passage of cultivation, preferably from passage 2, 3 or 4.

3. The method according to claim 1 or 2 **characterized in that** the primary keratinocytes are cultivated in a serum-free system before adding to the matrix.

4. The method according to claim 3 **characterized in that** the keratinocytes are cultivated in the absence of a feeder layer.

5. The method according to any one of the preceding claims whereby the primary fibroblasts are initially cultivated in a density of 1 x 10⁴ to 1 x 10⁶ cells/cm³ matrix.

6. The method according to any one of the preceding claims whereby the primary keratinocytes are initially cultivated in a density of 3 x 10⁵ to 7 x 10⁶ cells/cm² matrix surface.

7. The method according to any one of the preceding claims **characterized in that** the culture medium after addition of the primary fibroblast is a KCM medium as shown in table 1:
**Table 1: KCM medium**
| | | |
|---|---|---|
| Component | absolute | 500 ml |
| DMEM | 64.5% | 322.5 ml |
| Ham's F-12 | 21.5% | 107.5 ml |
| FCS | 10% | 50 ml |
| Hydrocortisone | 0.4 µg/ml | 5 ml |
| Cholera Toxin | 10-10 M | 50 µl |
| EGF | 10 ng/ml | 5 ml |
| Insulin | 5 µg/ml | 500 µl |
| Glutamine | 4 mM | 10 ml |
| Adenine | 0.00018 M | 500 µl |

8. The method according to any one of the preceding claims **characterized in that** no TGFβ1 is present in the culture medium at any process steps.

9. The method according to any one of the preceding claims for the generation of an in vitro skin model of a skin disease further comprising the steps of:
knocking down at least one disease related gene in the keratinocytes before adding said keratinocytes to the matrix.

10. The method according to claim 9 for the preparation of an in vitro skin model for autosomal recessive congenital ichthyosis comprising the step of knocking down at least one of the genes TGM1, ALOX2B, ALOXE3, ABCA12, ICHTHYIN(NIPAL4), and CYPF22.

11. A skin model obtainable according to any one of claims 1 to 10.

12. A method of screening for candidates for treating keratinisation disorders, in particular, autosomal recessive congenital ichthyosis, comprising the steps of
a) providing a skin model according to claim 11;
b) contacting said skin model with a candidate substance;
c) assaying for changes in physiological or morphological properties of the skin model;
d) identifying candidates causing the change of physiological or morphological properties in the skin model, in particular, changes in the barrier function and permeability as well as changes in the morphological structure in the skin model.

13. The method of claim 12 wherein the permeability of the barrier is determined by measuring Lucifer yellow diffusion.

14. Method for determining the therapy of an individual suffering from keratinisation disorders comprising the step of
- providing a skin model according to claim 11 containing keratinocytes and fibroblasts derived from said individual,
- contacting the skin model with a potential therapeutical, and
- determining whether said therapeutical is able to alleviate the keratinisation disorder, preferably, without displaying any adverse side effects.

15. Use of a skin model according to claim 11 or a method for generating a skin model according to any one of claims 1 to 10 for determining adverse effects, in particular, toxicity, of compounds, in particular, pharmaceuticals.


SEQUENCE LISTING

<110> Universität Köln

<120> Method for the generation of a skin model and skin model

<130> 4677-001 EP-1

<160> 50

<170> PatentIn version 3.3

<210> 1
<211> 18
<212> DNA
<213> Artificial

<220>
<223> primer

<400> 1
acccgagggc aagatgat 18

<210> 2
<211> 18
<212> DNA
<213> Artificial

<220>
<223> Primer

<400> 2
gcaggaagat ggggcaat 18

<210> 3
<211> 18
<212> DNA
<213> Artificial

<220>
<223> primer

<400> 3
aagcttcgag ggctgttg 18

<210> 4
<211> 23
<212> DNA
<213> Artificial

<220>
<223> primer

<400> 4
caccagtgct ctgtgacata ctt 23

<210> 5
<211> 21
<212> DNA
<213> Artificial

<220>
<223> primer

<400>  5
aggggaagac cttggacttt a                                                21

<210>  6
<211>  21
<212>  DNA
<213>  Artificial

<220>
<223>  primer

<400>  6
cgtctgacag ttcctttcca t                                                21

<210>  7
<211>  19
<212>  DNA
<213>  Artificial

<220>
<223>  primer

<400>  7
caacaccagc tgcgagaac                                                   19

<210>  8
<211>  20
<212>  DNA
<213>  artificial

<220>
<223>  primer

<400>  8
tgacaatctg gcacaggact                                                  20

<210>  9
<211>  21
<212>  DNA
<213>  artificial

<220>
<223>  primer

<400>  9
tgaaccatga ttctgtctgg a                                                21

<210>  10
<211>  18
<212>  DNA
<213>  artificial

<220>
<223>  primer

<400>  10
acctgccacc catcaaag                                                    18

<210>  11
<211>  20
<212>  DNA
<213>  artificial

<220>
<223>  primer

<400>  11
actgcactgg ccttgatagg                                                    20


<210>  12
<211>  18
<212>  DNA
<213>  artificial

<220>
<223>  primer

<400>  12
tcgacttcgg atccatgc                                                      18


<210>  13
<211>  21
<212>  DNA
<213>  artificial

<220>
<223>  primer

<400>  13
tgcgagtact caacaccaac a                                                  21


<210>  14
<211>  18
<212>  DNA
<213>  artificial

<220>
<223>  primer

<400>  14
gcatatcttc ggcccaca                                                      18


<210>  15
<211>  25
<212>  RNA
<213>  artificial

<220>
<223>  primer

<400>  15
agaaggccuc agcaaugagg ugugu                                              25


<210>  16
<211>  25
<212>  RNA
<213>  artificial

<220>
<223>  primer

<400>  16
acacaccuca uugcugaggc cuucu                                              25

<210>  17
<211>  25
<212>  RNA
<213>  artificial

<220>
<223>  primer

<400>  17
aaagugguuc agcagcugcu uaugg                                          25


<210>  18
<211>  25
<212>  RNA
<213>  artificial

<220>
<223>  primer

<400>  18
ccauaagcag cugcugaacc acuuu                                          25


<210>  19
<211>  25
<212>  RNA
<213>  artificial

<220>
<223>  primer

<400>  19
uaguugcagu accaaggguc cuugg                                          25


<210>  20
<211>  25
<212>  RNA
<213>  artificial

<220>
<223>  primer

<400>  20
ccaaggaccc uugguacugc aacua                                          25


<210>  21
<211>  25
<212>  RNA
<213>  artificial

<220>
<223>  primer

<400>  21
uacuuugucg ugaaggucuu auggc                                          25


<210>  22
<211>  26
<212>  RNA
<213>  artificial

<220>
<223>  primer

<400>  22
gccauaagga ccuucacgac aaagua                                          26


<210>  23
<211>  25
<212>  RNA
<213>  artificial

<220>
<223>  primer

<400>  23
agaaauuggu guaggugaag ugggc                                           25


<210>  24
<211>  25
<212>  RNA
<213>  artificial

<220>
<223>  primer

<400>  24
gcccacuuca ccuacaccaa uuucu                                           25


<210>  25
<211>  25
<212>  RNA
<213>  artificial

<220>
<223>  primer

<400>  25
uugcuaacca accagaagag gagga                                           25


<210>  26
<211>  25
<212>  RNA
<213>  artificial

<220>
<223>  primer

<400>  26
uccuccucuu cugguugguu agcca                                           25


<210>  27
<211>  25
<212>  RNA
<213>  artificial

<220>
<223>  primer

<400>  27
aauugaacag cauccaagag auccc                                           25


<210>  28
<211>  25
<212>  RNA
<213>  artificial

<220>
<223>  primer

<400>  28
gggaucucuu ggaugcuguu caauu                                          25


<210>  29
<211>  25
<212>  RNA
<213>  artificial

<220>
<223>  primer

<400>  29
ugaccaagca gaugauuccu uuggg                                          25


<210>  30
<211>  25
<212>  RNA
<213>  artificial

<220>
<223>  primer

<400>  30
cccaaaggaa ucaucugcuu gguca                                          25


<210>  31
<211>  25
<212>  RNA
<213>  artificial

<220>
<223>  primer

<400>  31
aacauaucaa gggagaccgc ugagc                                          25


<210>  32
<211>  25
<212>  RNA
<213>  artificial

<220>
<223>  primer

<400>  32
gcucagcggu cucccuugau auguu                                          25


<210>  33
<211>  25
<212>  RNA
<213>  artificial

<220>
<223>  primer

<400>  33
aaagcaugca gcaugaacac gccca                                          25

<210>  34
<211>  25
<212>  RNA
<213>  artificial

<220>
<223>  primer

<400>  34
ugggcguguu caugcugcau gcuuu                                          25


<210>  35
<211>  25
<212>  RNA
<213>  artificial

<220>
<223>  primer

<400>  35
aggaauggau uauaaauacu uuggg                                          25


<210>  36
<211>  25
<212>  RNA
<213>  artificial

<220>
<223>  primer

<400>  36
cccaaaguau uuauaaucca uuccu                                          25


<210>  37
<211>  25
<212>  RNA
<213>  artificial

<220>
<223>  primer

<400>  37
agcagguuca gacucucccu cagga                                          25


<210>  38
<211>  25
<212>  RNA
<213>  artificial

<220>
<223>  primer

<400>  38
uccugaggga gagucugaac cugcu                                          25


<210>  39
<211>  25
<212>  RNA
<213>  artificial

<220>
<223>  primer

<400>  39
uauagaggua ggugaugucc ucccg                                          25


<210>  40
<211>  25
<212>  RNA
<213>  artificial

<220>
<223>  primer

<400>  40
cgggaggaca ucaccuaccu cuaua                                          25


<210>  41
<211>  25
<212>  RNA
<213>  artificial

<220>
<223>  primer

<400>  41
uucacuacua gcaugcccuc uggga                                          25


<210>  42
<211>  25
<212>  RNA
<213>  artificial

<220>
<223>  primer

<400>  42
uccgagaggg caugcuagua gugaa                                          25


<210>  43
<211>  25
<212>  RNA
<213>  artificial

<220>
<223>  primer

<400>  43
uucagauucu gcccacuggc cuuga                                          25


<210>  44
<211>  25
<212>  RNA
<213>  artificial

<220>
<223>  primer

<400>  44
ucaaggccag ugggcagaau cugaa                                          25


<210>  45
<211>  25
<212>  RNA
<213>  artificial

<220>
<223>  primer

<400>  45
uaaacaugag gccauugcuc uucuc                                          25


<210>  46
<211>  25
<212>  RNA
<213>  artificial

<220>
<223>  primer

<400>  46
gagaagagca auggccucau guuua                                          25


<210>  47
<211>  25
<212>  RNA
<213>  artificial

<220>
<223>  primer

<400>  47
uucagaagca ucugcacugu uauug                                          25


<210>  48
<211>  25
<212>  RNA
<213>  artificial

<220>
<223>  primer

<400>  48
caauaacagu gcagaugcuu cugaa                                          25


<210>  49
<211>  25
<212>  RNA
<213>  artificial

<220>
<223>  primer

<400>  49
aauaaucuca gcucuauuau caggg                                          25


<210>  50
<211>  25
<212>  RNA
<213>  artificial

<220>
<223>  primer

<400>  50
cccugauaau agagcugaga uuauu                                          25